# EUROPEAN PATENT APPLICATION

(11) **EP 2 596 762 A1**
(43) Date of publication of application: **29.05.2013**
(21) Application number: 12194664.4
(22) Date of filing: 28.11.2012
(51) Int. Cl.: A61F 2/04, A61F 2/07, A61F 2/82

(54) **Biodegradable stents having one or more coverings**

(30) Priority: 28.11.2011 US 201161564028 P
(71) Applicant: Cook Medical Technologies LLC, Bloomington, IN 47404 (US)
(72) Inventor: Toomey, Ciaran, County Cork (IE); Ryan, Michael, Limerick (IE)
(74) Representative: Garratt, Peter Douglas

(57) **Abstract**

The present embodiments provide a medical device comprising a stent framework having proximal and distal regions and a lumen extending therebetween, and which comprises a biodegradable material. A first covering is coupled to at least a portion of an outer surface of the stent framework. When the stent framework is in an expanded deployed configuration, at least a portion of the first covering is disposed adjacent to a target site and fluid flows through the lumen of the stent framework. Further, the stent framework comprises a material that biodegrades a predetermined time after the first covering achieves at least partial remodeling at the target site. In various embodiments, one or more second coverings may be disposed adjacent to the first covering and comprise a material that biodegrades before the stent framework biodegrades.

## Description

### PRIORITY CLAIM

This invention claims the benefit of priority of U.S. Provisional Application Serial No. 61/564,028, entitled "Biodegradable Stents Having One or More Coverings," filed November 28, 2011, the disclosure of which is hereby incorporated by reference in its entirety.

### BACKGROUND

The present embodiments relate generally to medical devices, and more particularly, to biodegradable stents having one or more coverings.

There are various instances in which it might be desirable or necessary to remodel a segment of a patient's tissue. As one example, it might be necessary to facilitate closure of an opening in a bodily wall that was formed intentionally or unintentionally.

An example of an unintentional opening is a tracheoesophageal fistula occurring between the esophagus and the trachea. Other examples include esophageal fistulae, leaks and perforations. The current treatment options for such conditions include placement of a partially or fully covered esophageal stent to seal off the entrance to the fistula, thereby inhibiting food and fluids within the esophagus from passing into the trachea, and also inhibiting air in the trachea from passing into the esophagus.

An intentional opening may be formed, for example, during surgical procedures such as translumenal procedures. In a translumenal procedure, one or more instruments may be inserted through a visceral wall, such as the esophageal wall. For example, it may be desirable to endoscopically retrieve a lymph node situated within the mediastinal cavity, or gain access through an opening in the esophagus to perform therapies or diagnostics in the thoracic cavity.

During a translumenal procedure, a closure instrument may be used to close the opening in the visceral wall. Various closure devices include suturing devices, t-anchors, clips, and other devices that may apply compressive forces. Depending on the structure comprising the opening, it may be difficult to adequately close the opening and prevent leakage of bodily fluids.

With regard to the esophagus in particular, certain closure devices that apply compressive forces may not be desirable as they may impact the structure of the passageway. Further, such devices may leave strictures from scarring that may cause complications. Moreover, it may be difficult to deploy various closure devices or perform suturing in the esophagus. Further, even if the above techniques adequately treat the target tissue, e.g., by ensuring closure of an opening without leakage, such techniques may not promote remodeling of tissue over time, and in certain instances, it may not be desirable to permanently leave certain components within the passageway.

### SUMMARY

The present embodiments provide a medical device comprising a stent framework having proximal and distal regions and a lumen extending therebetween, and which comprises a biodegradable material. A first covering is coupled to at least a portion of an outer surface of the stent framework. When the stent framework is in an expanded deployed configuration, at least a portion of the first covering is disposed adjacent to a target site and fluid flows through the lumen of the stent framework. Further, the stent framework comprises a material that biodegrades a predetermined time after the first covering achieves at least partial remodeling at the target site.

In various embodiments, one or more second coverings may be disposed adjacent to the first covering, or overlapping at least a portion of the first covering. For example, a proximal second covering may be disposed proximal to the first covering and a distal second covering may be disposed distal to the first covering. In one embodiment, a portion of the stent framework disposed proximal to the proximal second covering remains uncovered, and a separate portion of the stent framework disposed distal to the distal second covering remains uncovered. The one or more second coverings may comprise a material that is dissolved, degraded or absorbed before the stent framework biodegrades.

The stent framework may comprise a central region disposed between the proximal and distal regions, and the first covering may be disposed within the central region of the stent framework. In one embodiment, the proximal and distal regions of the stent framework each comprise a greater outer diameter relative to an outer diameter of the central region.

In one embodiment, the first covering comprises small intestinal submucosa that is left in the passageway to promote tissue ingrowth. The first covering may cover only a selected portion of the outer surface of the stent framework. Further, the first covering may be coupled to the stent framework using at least one temporary connector, which in one embodiment comprises at least one resorbable suture.

Advantageously, the first covering promotes site-appropriate tissue remodeling to facilitate closure of an opening, resulting in a remodeled tissue segment that facilitates closure of the opening and further remodeling of the bodily lumen over time. The one or more second coverings may be dissolved, degraded or absorbed after a sufficient time to allow the first covering to promote closure of the openings, and further, the stent framework may be dissolved, degraded or absorbed after the second coverings have been dissolved, degraded or absorbed. There are therefore no long-term forces imposed upon the bodily lumen wall, and the inner diameter and structure of the bodily lumen is not impacted.

Other systems, methods, features and advantages of the invention will be, or will become, apparent to one with skill in the art upon examination of the following figures and detailed description. It is intended that all such additional systems, methods, features and advantages be within the scope of the invention, and be encompassed by the following claims.

### BRIEF DESCRIPTION OF THE DRAWINGS

The invention can be better understood with reference to the following drawings and description. The components in the figures are not necessarily to scale, emphasis instead being placed upon illustrating the principles of the invention. Moreover, in the figures, like referenced numerals designate corresponding parts throughout the different views.

FIG. 1 is a side view of a coated stent according to a first embodiment.

FIG. 2 is schematic illustration of the coated stent of FIG. 1 disposed in an esophagus, with the esophagus being shown in side-section and the coated stent being shown from a side view for illustrative purposes.

FIGS. 3-5 are cross-sectional views of alternative embodiments of the coated stent of FIG. 1.

FIGS. 6-8 are side views of coated stents according to alternative embodiments.

### DETAILED DESCRIPTION OF THE PREFERRED EMBODIMENTS

In the present application, the term "proximal" refers to a direction that is generally towards a physician during a medical procedure, while the term "distal" refers to a direction that is generally towards a target site within a patent's anatomy during a medical procedure.

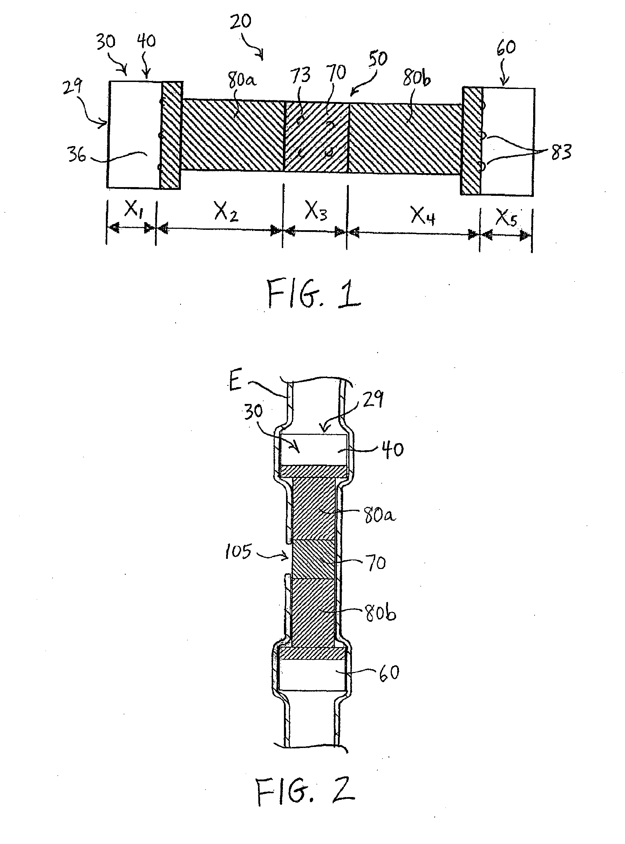

Referring to FIGS. 1-2, a first embodiment of a coated stent 20 is shown. The coated stent 20 comprises a stent framework 30 having a proximal region 40, a central region 50, and a distal region 60. The coated stent 20 further comprises at least one section having a first covering and at least one section having a second covering. In the example of FIGS. 1-2, the coated stent 20 comprises a first section having a first covering 70, and other sections having proximal and distal second coverings 80a and 80b, wherein the first covering 70 is disposed between the proximal and distal second coverings 80a and 80b. In alternative embodiments, the first covering 70 may be disposed at least partially on top of the proximal and distal second coverings 80a and 80b, or vice versa, or in other embodiments the proximal and distal second coverings 80a and 80b may comprise a unitary second covering that is disposed above or below the first covering 70.

The coated stent 20 has a delivery state that is suitable for insertion into a target duct or vessel of a patient, and an expanded deployed state as shown in FIGS. 1-2. A lumen 29 is formed between the proximal region 40, the central region 50, and the distal region 60 of the stent framework 30. In the expanded deployed state, the coated stent 20 has structural characteristics that are suitable for a particular application, such as radial force requirements to maintain patency within a vessel or duct.

The stent framework 30 provides the expansion characteristics of the coated stent 20, and may comprise self-expanding or balloon expandable characteristics to achieve the expanded deployed state in FIGS. 1-2. The first covering 70, and the second covering 80a and 80b, may be coated onto an exterior surface of the stent framework 30 as described further below.

The stent framework 30 may comprise any suitable shape, including a plurality of strut segments that are capable of expansion to the deployed state in FIGS. 1-2. In one example, strut segments of the stent framework 30 may comprise one or more zig-zag shaped segments. The stent framework 30 alternatively may comprise a pattern of interconnected struts, including diamond or other shapes as generally known in the art. The stent framework 30 may be made from a woven wire structure, a laser-cut cannula, individual interconnected rings, or any other type of stent framework that is known in the art.

Moreover, the stent framework 30 preferably is formed (and preferably wholly formed) from a biodegradable material. In one non-limiting example, the stent framework 30 comprises a dissolvable metal that degrades over a period of time. The stent framework 30 may be made from dissolvable metals that are biased, such that they may be restrained by a delivery device prior to deployment, but are inclined to return to their relaxed, expanded configuration shown in FIGS. 1-2 upon deployment. In one embodiment, the stent framework 30 comprises polydioxanone. In alternative embodiments, stent materials may comprise polyglycolic acid, polylactic acid, polycaprolactone, and magnesium. Such a listing is not exhaustive and it will be appreciated that different materials other than those listed may be used.

In the expanded deployed state of FIGS. 1-2, the proximal and distal regions 40 and 60 comprise cuff shapes having outer diameters greater than an outer diameter of the central region 50. Such a shape may be beneficial in certain applications, for example, as the cuff shapes may act as anti-migration features to resist the effects of peristalsis. Optionally, a smooth taper may be provided between the proximal region 40 and the central region 50, and/or between the central region 50 and the distal region 60, to reduce the steps depicted in FIGS. 1-2. In still further embodiments, the proximal region 40, the central region 50, and the distal region 60 each may comprise a substantially identical outer diameter, such that the outer diameter is substantially the same along the longitudinal length of the coated stent 20. Alternatively, the central region 50 may bow radially outward relative to the proximal and distal regions 40 and 60. In short, various combinations of outer diameter shapes of the coated stent 20 may be provided.

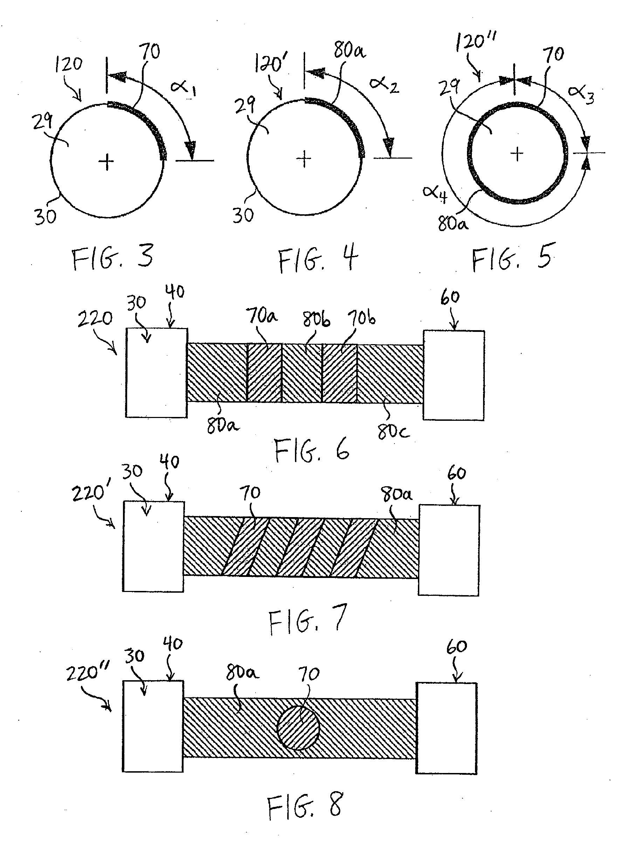

Referring still to FIGS. 1-2, the first covering 70 may be disposed to surround at least a portion of an outer surface of the stent framework 30. In the example shown, the first covering 70 covers only a central portion of the outer surface of the stent framework 30. Alternatively, the first covering 70 may cover other regions of the outer surface of the stent framework 30, as depicted in the examples of FIGS. 6-8 below.

In certain embodiments, the first covering 70 will be comprised of a remodelable material. Particular advantage can be provided by devices that incorporate a remodelable collagenous material. Such remodelable collagenous materials, whether reconstituted or naturally-derived, can be provided, for example, by collagenous materials isolated from a warm-blooded vertebrate, especially a mammal. Such isolated collagenous material can be processed so as to have remodelable, angiogenic properties and promote cellular invasion and ingrowth. Remodelable materials may be used in this context to stimulate ingrowth of adjacent tissues into an implanted construct such that the remodelable material gradually breaks down and becomes replaced by new patient tissue so as to generate a new, remodeled tissue structure.

Suitable remodelable materials of the first covering 70 can be provided by collagenous extracellular matrix (ECM) materials possessing biotropic properties. For example, suitable collagenous materials include ECM materials such as those comprising submucosa, renal capsule membrane, dermal collagen, dura mater, pericardium, fascia lata, serosa, peritoneum or basement membrane layers, including liver basement membrane. Suitable submucosa materials for these purposes include, for instance, intestinal submucosa including small intestinal submucosa, stomach submucosa, urinary bladder submucosa, and uterine submucosa. Collagenous matrices comprising submucosa (potentially along with other associated tissues) useful in the present invention can be obtained by harvesting such tissue sources and delaminating the submucosa-containing matrix from smooth muscle layers, mucosal layers, and/or other layers occurring in the tissue source. For additional information as to some of the materials useful in the present invention, and their isolation and treatment, reference can be made, for example, to U.S. Patent Nos. 4,902,508, 5,554,389, 5,993,844, 6,206,931, and 6,099,567.

Remodelable ECM tissue materials harvested as intact sheets from a mammalian source and processed to remove cellular debris advantageously retain at least a portion of and potentially all of the native collagen microarchitecture of the source extracellular matrix. This matrix of collagen fibers provides a scaffold to facilitate and support tissue ingrowth, particularly in bioactive ECM implant materials, such as porcine small intestinal submucosa or SIS (Surgisis® Biodesign™, Cook Medical, Bloomington IN), that are processed to retain an effective level of growth factors and other bioactive constituents from the source tissue. In this regard, when an inventive construct incorporates this sort of material, cells will invade the remodelable material upon implantation eventually leading to the generation of a newly-remodeled, functional tissue structure.

Submucosa-containing or other ECM tissue used in the invention is preferably highly purified, for example, as described in U.S. Patent No. 6,206,931 to Cook et al. Thus, preferred ECM material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 may be characteristic of any ECM tissue used in the present invention.

A typical layer thickness for an as-isolated submucosa or other ECM tissue layer used in the invention ranges from about 50 to about 250 microns when fully hydrated, more typically from about 50 to about 200 microns when fully hydrated, although isolated layers having other thicknesses may also be obtained and used. These layer thicknesses may vary with the type and age of the animal used as the tissue source. As well, these layer thicknesses may vary with the source of the tissue obtained from the animal source. In a dry state, a typical layer thickness for an as-isolated submucosa or other ECM tissue layer used in the invention ranges from about 30 to about 160 microns when fully dry, more typically from about 30 to about 130 microns when fully dry.

Suitable bioactive agents may include one or more bioactive agents native to the source of the ECM tissue material. For example, a submucosa or other remodelable ECM tissue material may retain one or more growth factors such as but not limited to basic fibroblast growth factor (FGF-2), transforming growth factor beta (TGF-beta), epidermal growth factor (EGF), cartilage derived growth factor (CDGF), and/or platelet derived growth factor (PDGF). As well, submucosa or other ECM materials when used in the invention may retain other native bioactive agents such as but not limited to proteins, glycoproteins, proteoglycans, and glycosaminoglycans. For example, ECM materials may include heparin, heparin sulfate, hyaluronic acid, fibronectin, cytokines, and the like. Thus, generally speaking, a submucosa or other ECM material may retain one or more bioactive components that induce, directly or indirectly, a cellular response such as a change in cell morphology, proliferation, growth, protein or gene expression.

Submucosa-containing or other ECM materials of the present invention can be derived from any suitable organ or other tissue source, usually sources containing connective tissues. The ECM materials processed for use in the invention will typically include abundant collagen, most commonly being constituted at least about 80% by weight collagen on a dry weight basis. Such naturally-derived ECM materials will for the most part include collagen fibers that are non-randomly oriented, for instance occurring as generally uniaxial or multi-axial but regularly oriented fibers. When processed to retain native bioactive factors, the ECM material can retain these factors interspersed as solids between, upon and/or within the collagen fibers. Particularly desirable naturally-derived ECM materials for use in the invention will include significant amounts of such interspersed, non-collagenous solids that are readily ascertainable under light microscopic examination with appropriate staining. Such non-collagenous solids can constitute a significant percentage of the dry weight of the ECM material in certain inventive embodiments, for example at least about 1%, at least about 3%, and at least about 5% by weight in various embodiments of the invention.

The submucosa-containing or other ECM material used in the present invention may also exhibit an angiogenic character and thus be effective to induce angiogenesis in a host engrafted with the material. In this regard, angiogenesis is the process through which the body makes new blood vessels to generate increased blood supply to tissues. Thus, angiogenic materials, when contacted with host tissues, promote or encourage the formation of new blood vessels into the materials. Methods for measuring in vivo angiogenesis in response to biomaterial implantation have recently been developed. For example, one such method uses a subcutaneous implant model to determine the angiogenic character of a material. See, C. Heeschen et al., Nature Medicine 7 (2001), No. 7, 833-839. When combined with a fluorescence microangiography technique, this model can provide both quantitative and qualitative measures of angiogenesis into biomaterials. C. Johnson et al., Circulation Research 94 (2004), No. 2, 262-268.

Further, in addition or as an alternative to the inclusion of such native bioactive components, non-native bioactive components such as those synthetically produced by recombinant technology or other methods (e.g., genetic material such as DNA), may be incorporated into an ECM material. These non-native bioactive components may be naturally-derived or recombinantly produced proteins that correspond to those natively occurring in an ECM tissue, but perhaps of a different species. These non-native bioactive components may also be drug substances. Illustrative drug substances that may be added to materials include, for example, anti-clotting agents, e.g. heparin, antibiotics, antiinflammatory agents, thrombus-promoting substances such as blood clotting factors, e.g., thrombin, fibrinogen, and the like, and anti-proliferative agents, e.g. taxol derivatives such as paclitaxel. Such non-native bioactive components can be incorporated into and/or onto ECM material in any suitable manner, for example, by surface treatment (e.g., spraying) and/or impregnation (e.g., soaking), just to name a few. Also, these substances may be applied to the ECM material in a premanufacturing step, immediately prior to the procedure (e.g., by soaking the material in a solution containing a suitable antibiotic such as cefazolin), or during or after engraftment of the material in the patient.

Inventive devices can incorporate xenograft material (i.e., cross-species material, such as tissue material from a non-human donor to a human recipient), allograft material (i.e., interspecies material, with tissue material from a donor of the same species as the recipient), and/or autograft material (i.e., where the donor and the recipient are the same individual). Further, any exogenous bioactive substances incorporated into an ECM material may be from the same species of animal from which the ECM material was derived (e.g. autologous or allogenic relative to the ECM material) or may be from a different species from the ECM material source (xenogenic relative to the ECM material). In certain embodiments, ECM material will be xenogenic relative to the patient receiving the graft, and any added exogenous material(s) will be from the same species (e.g. autologous or allogenic) as the patient receiving the graft. Illustratively, human patients may be treated with xenogenic ECM materials (e.g. porcine-, bovine- or ovine-derived) that have been modified with exogenous human material(s) as described herein, those exogenous materials being naturally derived and/or recombinantly produced.

The first covering 70 may be coupled to at least a portion of an outer surface 36 of the stent framework 30. In one embodiment, the first covering 70 is coupled to the outer surface 36 of the stent framework 30 using at least one temporary connector 73. For example, the temporary connector 73 may comprise a biologically resorbable, degradable, dissolvable or erodible material. After the particular process of resorption, degradation, dissolution and/or erosion has been completed or substantially completed, the connection between the first covering 70 and the stent framework 30 is weakened or eliminated. In one example, a plurality of temporary connectors 73 are provided in the form of resorbable sutures. In alternative embodiments, the first covering 70 may be coupled to the outer surface 36 of the stent framework 30 using a biodegradable adhesive, or alternatively using a dipping process to achieve a coupling effect. As noted above, the first covering 70 may be disposed to surround only one or more selected portions of the outer surface 36 of the stent framework 30, for example, those portions that are expected to be adjacent to a portion of a passageway for which tissue remodeling is desired.

Referring still to FIGS. 1-2, second coverings 80a and 80b also may be disposed to surround the outer surface 36 of the stent framework 30. The second coverings 80a and 80b may be made from a degradable, dissolvable or absorbable material that is substantially impermeable to acids, food and the like. By way of example and without limitation, the second coverings 80a and 80b may comprise polyglycolic acid, polylactic acid, polydioxanone, or other materials. Like the first covering 70, the second coverings 80a and 80b also may be coupled to at least a portion of the outer surface 36 of the stent framework 30 using at least one temporary connector 83 such as a resorbable suture, or may be coupled using a biodegradable adhesive, spraying or dipping process. In certain alternatives, however, at least one of the second coverings 80a and 80b may be positioned at least partially on the inner surface of the stent framework 30.

In one exemplary application shown in FIG. 2, the first covering 70 may promote site-appropriate tissue remodeling to facilitate closure of one or more openings 105. Over a period of time, a portion of the stent framework 30 and the second coverings 80a and 80b may dissolve, degrade or be absorbed, leaving the first covering 70 within the passageway, as explained in further detail below. During treatment, as the first covering 70 promotes site-appropriate tissue remodeling to facilitate closure of one or more openings 105, the second coverings 80a and 80b may effectively isolate at least a portion of the first covering 70 to facilitate the smart or "site-appropriate" tissue remodeling by the first covering 70 during the healing process.

Additionally, the stent framework 30 provides structural stability to hold the first covering 70 in place throughout the healing process, prior to the stent framework dissolving, degrading, or being absorbed. The stent framework 30 also may hold the second coverings 80a and 80b in place prior to the stent framework dissolving, degrading, or being absorbed. In one embodiment, the second coverings 80a and 80b are dissolved, degraded or absorbed after a sufficient time to allow the first covering 70 to promote closure of the openings 105, and further, the stent framework 30 is dissolved, degraded or absorbed after the second coverings 80a and 80b have been dissolved, degraded or absorbed. Alternatively, if the stent framework dissolves, degrades or is absorbed prior to the second coverings 80a and 80b, the second coverings 80a and 80b may fall off and be absorbed by the stomach or passed through the bodily system.

The temporary connectors 73 coupling the first covering 70 to the stent framework 30 may be dissolved, degraded, absorbed or eroded after a sufficient time that allows the first covering 70 to promote closure of the openings 105. Further, the temporary connectors 83 coupling the second coverings 80a and 80b to the stent framework 30 may be dissolved, degraded, absorbed or eroded around the same time that the stent framework 30 is dissolved, degraded or absorbed.

The features of the coated stent 20 of FIGS. 1-2 are generally divided into different lengths **X₁** through **X₅,** as shown in FIG. 1. There is an uncoated length **X₁** of the stent framework 30 disposed at a proximal end of the proximal region 40, and further there is an uncoated length **X₅** of the stent framework 30 disposed at a distal end of the distal region 60, as shown in FIG. 1. Being part of the flared or cuff-shaped proximal and distal regions 40 and 60, the uncoated lengths **X₁** and **X₅** of the stent framework 30 engage an inner wall of the bodily lumen, and tissue ingrowth may be achieved in these uncoated regions around struts of the stent framework 30 to thereby further stabilize the position of the coated stent 20 relative to the bodily lumen.

In the embodiment of FIGS. 1-2, the first covering 70 comprises an axial length **X₃** that is greater than a length of the opening 105 to thereby overlay the entirety of the opening 105, as depicted in FIG. 2. The proximal second covering 80a extends a length **X₂** between the first covering 70 and onto the proximal region 40, and the distal second covering 80b extends a length **X₄** between the first covering 70 and onto the distal region 60. In this manner, the proximal and distal second coverings 80a and 80b extend from the first covering 70 and onto the flared or cuff-shaped proximal and distal regions 40 and 60 to help reduce the possibility of fluids being transferred through the opening 105.

In the exemplary application of FIG. 2, a portion of an esophagus **E** is shown, and the opening 105 may arise as part of a tracehoesophageal fistula. Alternatively, in a medical procedure, it may become necessary or desirable to create the opening 105 in the esophagus **E.** For example, in one procedure, it may be desirable to endoscopically retrieve a lymph node situated within the mediastinal cavity, such as a malignant node. In other procedures, it may be desirable to gain access through the opening 105 in the esophagus **E** to perform therapies or diagnostics in the thoracic cavity using a translumenal approach.

With the opening 105 in the esophagus **E** being present, the coated stent 20 of FIG. 1 may be deployed. The coated stent 20 may be deployed in the esophagus **E** using a suitable stent deployment system. One exemplary system is shown in U.S. Published Application No. 2009/0281610 Al ("the '610 publication"), which is incorporated by reference in its entirety. While the '610 publication describes one system for delivering and deploying the coated stent 20 described herein, other suitable delivery and deployment systems may be used to deliver the coated stent 20 in the esophagus **E** in accordance with the techniques described herein. Using such a suitable delivery system, the stent framework 30 achieves the expanded deployed configuration such that fluid flows through the lumen 29 of the coated stent 20.

As shown in FIG. 2, after deployment the coated stent 20 is positioned within the esophagus **E** such that the first covering 70 is aligned with the opening 105. As noted above, the axial length **X₃** of the first covering may be greater than a length of the opening 105 to thereby overlay the entirety of the opening 105.

Preferably, one or more regions of the stent comprise at least one radiopaque marker to allow a physician to overlay the first covering 70 with the opening 105, in addition to readily identifying the proximal and distal regions 40 and 60 during placement of the coated stent 20. Such radiopaque markers may be formed from a biodegradable material and may be coupled to struts of the stent framework 30 at desired imaging locations.

Upon deployment of the coated stent 20 as shown in FIG. 2, the flared or cuff-shaped proximal and distal regions 40 and 60 engage healthy portions of the esophagus **E** at locations proximal and distal to the opening 105. Optionally, resorbable barbs of the proximal and distal regions 40 and 60 may engage an inner surface of the esophagus **E.** Further, the uncoated lengths **X₁** and **X₅** of the stent framework 30 engage the inner wall of the esophagus **E,** and tissue ingrowth may be achieved in these uncoated regions, around struts of the stent framework 30, to thereby further stabilize the position of the coated stent 20 within the esophagus **E.**

Further, upon deployment of the coated stent 20, the second coverings 80a and 80b provide substantially impermeable proximal and distal barriers, respectively, to help protect the first covering 70 from substances such as acids, food and the like, during healing of the opening 105. Additionally, the second coverings 80a and 80b may reduce the likelihood of acids, food and the like exiting around the coated stent 20 and through the opening 105 into the peritoneum, or trachea when a tracehoesophageal fistula is being treated.

As noted above, the second coverings 80a and 80b are dissolved, degraded or absorbed after a sufficient time to allow the first covering 70 to promote closure of the openings 105, and further, the stent framework 30 is dissolved, degraded or absorbed after the second coverings 80a and 80b have been dissolved, degraded or absorbed. There are therefore no long-term forces imposed upon the esophageal wall, and the inner diameter and structure of the esophagus is not impacted. Further, since the stent framework 30, second coverings 80a and 80b, and any temporary connectors 73 and 83 are dissolved, they need not be left within the patient's body and no secondary procedures are needed.

Referring now to FIGS. 3-5, cross-sectional views are shown of alternative embodiments of the coated stent of FIG. 1. In FIG. 3, an alternative coated stent 120 comprises a first covering 70 extending an angle α**₁** around the circumference of the stent framework 30. While the angle α**₁** is shown as being around 90 degrees, it may range from between about 5 degrees to about 360 degrees around the circumference of the stent framework 30, and the remainder of the circumferences of the stent framework 30 is uncovered. In FIG. 4, an alternative coated stent 120' comprises a second covering 80a extending an angle α**₂** around the circumference of the stent framework 30. The angle α**₂** is shown as being around 90 degrees, although it may range from between about 5 degrees to about 360 degrees around the circumference of the stent framework 30. In FIG. 5, an alternative coated stent 120" comprises a first covering 70 extending an angle α**₃** around the circumference of the stent framework 30, and a second covering 80a extending an angle α**₄** around the circumference of the stent framework 30. While the angle α**₃** is shown as being around 90 degrees and the angle α**₄** is shown as being around 270 degrees, the relative circumferential coverage of the first covering 70 and the second covering 80a may vary. The combinations shown in FIGS. 3-5 advantageously allow for variation in the circumferential provision of the bare stent framework 30, the first covering 70 and/or the second covering 80a, each of which provides different features as described above.

Referring now to FIGS. 6-8, side views of alternative coated stents are shown and described. In FIG. 6, an alternative coated stent 220 comprises a pair of separated first coverings 70a and 70b, and three separated second coverings 80a, 80b and 80c. One first covering 70a is disposed between the second coverings 80a and 80b, while the another first covering 70b is disposed between the second coverings 80b and 80c, as shown in FIG. 6. In the embodiment of FIG. 7, an alternative coated stent 220' comprises the first covering 70 having a helical shape that is wrapped adjacent to helical segments of the second covering 80a. In FIG. 8, an alternative coated stent 220" comprises a circular patch of the first covering 70 surrounded by the second covering 80a. Various other combinations of positioning of the uncovered stent framework 30, the one or more first coverings 70, and the one or more second coverings 80 are possible, both along the axial length of the coated stent and around its circumference.

It should be noted that while the exemplary embodiments herein depict treatment of an opening formed in the esophagus, the coated stents 20, 120, 120', 120", 220, 220' and 220", as well as methods described herein, may be used to treat any particular defect or condition in any vessel, duct, or other passageway. Further, in alternative embodiments, the second coverings 80a and 80 may be omitted and only the first covering 70 may be coupled to the stent framework 30.

While various embodiments of the invention have been described, the invention is not to be restricted except in light of the attached claims and their equivalents. Moreover, the advantages described herein are not necessarily the only advantages of the invention and it is not necessarily expected that every embodiment of the invention will achieve all of the advantages described.

## Claims

1. A medical device, comprising:
a stent framework having proximal and distal regions and a lumen extending therebetween, wherein the stent framework comprises a biodegradable material; and
a first covering coupled to at least a portion of an outer surface of the stent framework,
wherein, when the stent framework is in an expanded deployed configuration, at least a portion of the first covering is disposed adjacent to a target site and fluid flows through the lumen of the stent framework, and
wherein the stent framework comprises or consists of a material that is configured to biodegrade a predetermined time after the first covering achieves at least partial remodeling at the target site.

2. The medical device of claim 1 further comprising at least one second covering coupled to at least a portion of an outer surface of the stent framework and disposed adjacent to the first covering.

3. The medical device of claim 1 or claim 2 comprising proximal and distal second coverings coupled to at least a portion of an outer surface of the stent framework, wherein the proximal second covering is disposed proximal to the first covering and the distal second covering is disposed distal to the first covering.

4. The medical device of claim 3 wherein a portion of the stent framework disposed proximal to the proximal second covering remains uncovered, and wherein a separate portion of the stent framework disposed distal to the distal second covering remains uncovered.

5. The medical device of any one of the preceding claims, wherein the stent framework comprises a material that biodegrades a predetermined time after the first covering achieves a full remodeling at the target site.

6. The medical device of any one of the preceding claims wherein the stent framework further comprises a central region disposed between the proximal and distal regions, wherein the first covering is disposed within the central region of the stent framework, and wherein the proximal and distal regions each comprise a greater outer diameter relative to an outer diameter of the central region.

7. The medical device of any one of the preceding claims wherein the first covering is coupled to the stent framework using at least one temporary connector that comprises at least one of a biologically resorbable, degradable, dissolvable or erodible material.

8. The radical device of any one of the preceding claims wherein the first covering comprises or consists of a removable material, a remodelable collagenous material, a collagenous extracellular matrix material, or a submucosa.

9. The medical device of any one of the preceding claims wherein the first covering comprises small intestinal submucosa.

10. The medical device of any one of the preceding claims wherein the first covering covers only a selected portion of the outer surface of the stent framework.

11. A medical device according to any one of the preceding claims, comprising:
a second covering coupled to at least a portion of an outer surface of the stent framework and disposed adjacent to the first covering,
wherein the second covering is configured to biodegrade a predetermined time after the first covering achieves at least partial remodeling at the target site, and
wherein the second covering comprises a material that biodegrades before the stent framework biodegrades.
